# EUROPÄISCHE PATENTANMELDUNG

(11) **EP 4 098 677 A1**
(43) Veröffentlichungstag der Anmeldung: **07.12.2022**
(21) Anmeldenummer: 21177501.0
(22) Anmeldetag: 02.06.2021
(51) Int. Cl.: C08G 18/00, C10B 53/07, C10B 57/12

(54) **PYROLYSE VON MATERIAL MIT POLYURETHAN-VERBINDUNG ZUR WIEDERGEWINNUNG VON ROHSTOFFEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Für die gewerbliche Ausführung einer Pyrolyse wurde ein Verfahren gemäß Anspruch 1 sowie darin nutzbare Pyrolysevorrichtungen für die Pyrolyse von Pyrolysegut umfassend Polyurethan-haltiges Material, bereitgestellt. Damit wird auch bei größeren Mengen von Pyrolysegut eine Menge an Pyrolyseprodukt erhalten, welches für die Synthese von Polyurethan-haltigem Material wiederverwertbare Spaltprodukte enthält.

## Beschreibung

Die Erfindung betrifft ein Pyrolyseverfahren zur thermischen Verwertung von Polyurethan-haltigem Material, eine entsprechende Verwendung einer Pyrolysevorrichtung sowie das Produkt der Pyrolyse, welches solche Rohstoffe enthält, die zur Herstellung von Polyurethan wiederverwertet werden können.

Polyurethan-haltiges Material findet als Polsterung oder als Isoliermaterialien im Kühl- und Baubereich Anwendung. Insbesondere schaumförmige Polyurethan-haltige Materialien werden z.B. als Polster, Matratze, Dämm- oder Isolationsmaterial genutzt. Zur Verwendung Polyurethan-haltiger Materialien als Isolierung, z.B. für Kühlschränke oder im Bau, wird überwiegend Polyurethanhartschaum eingesetzt.

Am Ende der Lebensdauer der Polyurethanmaterial-haltigen Produkte werden sie gegen neue Produkte ausgetauscht und in der Regel verschrottet. Die Gesamtmenge der resultierenden Kunststoffabfälle nimmt jedes Jahr zu. Etwa 60% der Gesamtmenge wird durch Verbrennen und auf der Deponie entsorgt. Bei der Verbrennung wird CO₂ in die Luft emittiert, das zu der globalen Erwärmung beiträgt. Plastikabfall auf den Deponien nimmt wegen seiner geringen Dichte ein großes Volumen ein und kann von dort auch zur allgemeinen Verschmutzung in Flüssen und Meeren beitragen. Aus diesem Grunde ist es wichtig, eine effiziente Recycling Methode zu entwickeln, mit der das Abfallproblem gelöst werden kann und auch gleichzeitig fossile Ressourcen eingespart werden können. Da der Anteil insbesondere der oben genannten Polyurethanhartschäume mit ca. 8 % des Polymermarktes nur einen kleinen Teil ausmacht, stand das Recycling von Polyurethanen zunächst nicht im Fokus der Entwicklungen. Nunmehr ist es jedoch aufgrund des weiter wachsenden Kunststoffmarktes wichtig, für alle Polymerklassen Recycling Technologien zu entwickeln, um sowohl die CO₂ Emission zu vermindern und fossile Energien einzusparen.

Die Verfahren für das Recycling von Kunststoffabfällen lassen sind grob in 3 Kategorien unterteilen:
(1) mechanisches Recycling: Hierbei werden die Kunststoffabfälle so wie sie sind wieder eingeschmolzen und können dann wiederverwendet werden. Dies ist für Polyurethan Elastomere nicht möglich.
(2) chemisches und thermochemisches Recycling: Hierbei werden die Kunststoffabfälle in Monomere depolymerisiert oder zu kleineren Molekülen zersetzt, um nützliche chemische Rohstoffe zu gewinnen und
(3) thermisches Recycling, wobei die Kunststoffabfälle hierin zu Abgas und Wärmeenergie umgesetzt werden.

Die aus dem thermochemischen Recycling gewonnenen chemischen Rohstoffe können zur Synthese neuer Kunstharze oder anderer chemischer Produkte verwertet werden.

Das thermochemische Recycling wird als Pyrolyse bezeichnet. In den meisten Fällen wird die Pyrolyse für Verpackungsmüll angewendet und daraus ein Pyrolyse-Öl gewonnen, das als eine Art recyceltes Naphtha in den bekannten Raffinerie Prozessen mit Cracker als Drop in Lösung verwendet wird. Über die Pyrolyse von Polyurethanen ist wenig bekannt.

Die Verwendung von Katalysatoren oder Additiven kann im Pyrolyse Prozess zur Erniedrigung der Betriebstemperatur, zur Verkürzung der Reaktionszeit, zur Erhöhung der Degradationseffizienz und zur Einschränkung der Produktverteilung führen, was den Prozess effizienter macht.

Kumagaia et al. (Journal of Analytical and Applied Pyrolysis 126 (2017) 337-345) beschreiben eine bei ca. 800 °C durchgeführte Pyrolyse von Weich und Hartschaum zu Isocyanaten, Diaminen und sehr vielen weiteren Fragmenten aus dem Polyol.

M. Blazsó et al. (J. Chromatogr. A 1271 (2013) 217- 220) konnten zeigen, dass man durch die Verwendung von einem basischen NH₄Y Zeolithen als Hauptprodukt THF aus dem Polyol erhält. Zwei weitere leicht zu identifizierende Produkte waren Anilin und 4-Methylaniline, die durch katalytische Zersetzung von MDI über dem NH₄Y Zeolithen entstehen.

In DE 2410505 C2 wird ein Verfahren zur thermischen Herstellung von Isocyanaten aus Urethanen bei 400°C bis 530 und Unterdruck beschrieben. Versuche mit Polyurethanen unter Normaldruck werden nicht erwähnt.

In DE 2362915 wird die Hydrolyse von Polyurethan Weichschaum zu Diaminen in einen Fließbett vorgeschlagen.

Die im Stand der Technik beschriebenen Pyrolyse-Verfahren eignen sich allesamt nicht für die industrielle bzw. gewerbliche Anwendung, da sie zumeist als Pyrolysen im Microgramm Maßstab für analytische Zecke vorgesehen sind. Die im Stand der Technik dargelegten Pyrolysen werden daher jeweils in nicht für die industrielle bzw. gewerbliche Anwendung geeigneten Pyrolysevorrichtungen ausgeführt.

Insbesondere für die Pyrolyse größerer Mengen an Polyurethan-haltigen Materialien, sind neue, selektivere Verfahren mit passenden Katalysatoren und Reaktoren nötig, mit deren Hilfe sich die Bildung von ungewünschten Nebenprodukten verringern lässt. Aus Polyurethan-haltigen Materialien soll gezielt Pyrolyseprodukt erhalten werden, das einem hohen Gehalt an für die Polyurethan-Synthese wieder verwertbaren Spaltprodukten, insbesondere aromatische Aminverbindungen wie z.B. Anilin, Toluidin, Methylendianilin (mMDA) oder polymerem Methylendianilin (pMDA), enthalten.

Es war daher die Aufgabe, für die gewerbliche Ausführung einer Pyrolyse ein Verfahren sowie darin nutzbare Pyrolysevorrichtungen für die Pyrolyse von Pyrolysegut, mindestens umfassend Polyurethan-haltiges Material bereitzustellen, mit dessen Einsatz auch bei größeren Mengen von Pyrolysegut eine Menge an Pyrolyseprodukt erhalten wird, das für die Synthese von Polyurethan-haltigem Material wiederverwertbare Spaltprodukte, bevorzugt in einer Menge von mehr als 40 Gew.-% bezogen auf das Gesamtgewicht an eingesetztem Polyurethan-haltigem Material, enthält.

Weiter war es eine untergeordnete Aufgabe, bei der Pyrolyse von auf alkylenverbrücktem, aromatischem Isocyanat (z.B. Methylendiphenylisocyanat (MDI) oder polymerem Methylendiphenyl-isocyanat (pMDI)) basierendem Polyurethan-haltigem Material möglichst solche aromatischen Amine zurückzuerhalten, die Struktureinheiten mit alkylenverbrückten Aromateinheiten enthalten.

Ein Gegenstand der vorliegenden Anmeldung ist deshalb ein Verfahren zur Pyrolyse, umfassend mindestens die folgenden Schritte
(a) Einbringung von Pyrolysegut, mindestens umfassend
   i) Material enthaltend mindestens eine polymere Verbindung mit mindestens einer Polyurethan-Struktureinheit der Formel (I), 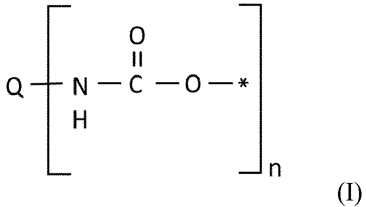 worin
      Q für einen Kohlenwasserstoffrest mit 8 bis 70 Kohlenstoffatomen, bevorzugt 10 bis 30 Kohlenstoffatomen, und
      n für eine Zahl von 2 bis 10, bevorzugt von 2 bis 6, besonders bevorzugt von 2 bis 4, steht
      und -* eine kovalente Bindung zum Polymerrückgrat bedeutet, und
   ii) mindestens einen, die thermische Zersetzung der besagten polymeren Verbindung beeinflussenden Katalysator,
   in einen Reaktor;
(b) Zersetzung zumindest des in Schritt (a) eingebrachten Materials des Pyrolyseguts in dem Reaktor bei einer Temperatur von 250°C bis 700°C, unter Erhalt von in der Gasphase befindlichem Produkt als Pyrolysat und von nicht in der Gasphase befindlichem Pyrolyserückstand, wobei
   (i) während der besagten Zersetzung die Menge an Sauerstoffgas im Reaktor höchstens 2,0 Vol.% bezogen auf das Gesamtvolumen der im Reaktor befindlichen Gase beträgt, und
   (ii) während der besagten Zersetzung das Pyrolysat aus dem Reaktor herausgeführt wird, und
   (iii) der besagte Pyrolyserückstand aus dem Reaktor herausgeführt wird;
(c) Abkühlung des herausgeführten Pyrolysates auf eine Temperatur von weniger als 250°C unter Erhalt von Pyrolyseprodukt, ausgewählt aus Pyrolysatkondensat, Pyrolysatsublimat oder einer Mischung daraus;
(d) optional Aufarbeitung des Pyrolyseprodukts.

Als "Pyrolysegut" wird die Gesamtheit der in den Reaktor zur Pyrolyse eingebrachten Stoffe bezeichnet, die dort in Abwesenheit von Sauerstoffgas oder in Gegenwart einer reduzierten Menge an Sauerstoffgas thermisch behandelt wird. Das Pyrolysegut ist vor der Einbringung in den Reaktor bevorzugt festförmig.

Unter "Pyrolysat" wird die Gesamtheit diejenigen durch Pyrolyse gebildeten Produkte verstanden, die sich unter den Bedingungen des Schrittes (b) (insbesondere als Gas und/oder als Aerosol) in der Gasphase des Reaktors befinden.

Unter "Pyrolyserückstand" wird die Gesamtheit derjenigen durch Pyrolyse gebildeten Stoffe und der sonstigen Rückstände des Pyrolyseguts verstanden, die sich unter den Bedingungen des Schrittes (b) nicht in der Gasphase des Reaktors befinden. Es sind solche Ausführungsformen des Verfahrens bevorzugt, die sich dadurch kennzeichnen, dass der Pyrolyserückstand im Reaktor festförmig ist.

Unter "Pyrolyseprodukt" wird die Gesamtheit derjenigen Produkte aus dem Pyrolysat verstanden, die in Schritt (c) bei der Abkühlung des Pyrolysates durch Kondensation und/oder Resublimation anfallen. Pyrolyseprodukt, das flüssig ist, wird auch als Pyrolyseöl bezeichnet.

Falls nicht explizit im Zusammenhang anders definiert, ist ein Stoff (z.B. Material, Pyrolysegut, Pyrolysat, Pyrolyseprodukt, Pyrolyserückstand) "flüssig", wenn er bei 20°C und 1013 mbar im flüssigen Aggregatzustand vorliegt. Falls nicht explizit im Zusammenhang anders definiert, ist ein Stoff (z.B. Material, Pyrolysegut, Pyrolysat, Pyrolyseprodukt, Pyrolyserückstand) "festförmig", wenn er bei 20°C und 1013 mbar im festen Aggregatzustand vorliegt. Falls nicht explizit im Zusammenhang anders definiert, ist ein Stoff (z.B. Material, Pyrolysegut, Pyrolysat, Pyrolyserückstand) "gasförmig", wenn er bei 20°C und 1013 mbar im als Gas vorliegt.

Ein Stoff ist "organisch", wenn seine chemische Struktur mindestens eine kovalente Kohlenstoff-Wasserstoff-Bindung enthält.

Bei den im Rahmen dieser Anmeldung für Polymere bzw. polymere Inhaltsstoffe angegebenen mittleren Molmassen handelt es sich - sofern nicht explizit anders gekennzeichnet - stets um gewichtsmittlere Molmassen Mw, die grundsätzlich mittels Gelpermeationschromatographie mit Hilfe eines RI-Detektors bestimmbar sind, wobei die Messung zweckmäßig gegen einen externen Standard erfolgt.

Ein "Reaktor" ist ein Volumen, in dem eine chemischen Umwandlung, z.B. eine thermische Zersetzung von Material aus dem Pyrolysegut, stattfindet. Dies kann für eine thermische Zersetzung beispielsweise das Volumen eines beheizten Gefäßes sein, in dem sich das Pyrolysegut befindet.

Es ist erfindungsgemäß vorteilhaft, das Pyrolysegut gemäß erfindungsgemäßem Verfahren in einen Reaktor einzubringen, der sich dadurch gekennzeichnet, dass er ausgewählt wird aus kontinuierlichem Rührkesselreaktor (CSTR), Festbettreaktor, Fließbettreaktor, Schneckenreaktor, Schneckenförderer Reaktor, Flugstromreaktor, Mitreißstromreaktor, Drehrohrreaktor, Fließbettreaktor, Schaufelreaktor. Insbesondere sind solche Reaktoren bevorzugt geeignet, in denen das Pyrolysegut kontinuierlich eingebracht werden kann und werden insbesondere ausgewählt aus Drehrohrreaktor, kontinuierlichem Rührkesselreaktor (CSTR), Festbettreaktor (insbesondere mit kontinuierlichem Bettaustausch (Schachtreaktor) mit innenliegendem Wärmetauscher, bevorzugt mit innenliegenden Wärmetauscherrohren, Schneckenreaktor, Schneckenfördererreaktor, Flugstromreaktor, Drehrohrreaktor oder Wirbelschichtreaktor. Ein ganz besonders bevorzugter Reaktor einer Ausführungsform des Verfahrens wird ausgewählt aus Schneckenreaktor, Drehrohrofen oder Wirbelbett. Weitere für das erfindungsgemäße Verfahren bevorzugte Reaktoren und deren Ausführungsformen werden im Rahmen der Ausführungsformen der erfindungsgemäßen Vorrichtung zur Pyrolyse, sowie im Rahmen einer Ausführungsform des Verfahrens mit Einsatz von Katalysator, beschrieben (*vide infra*).

Das in den Reaktor eingebrachte Pyrolysegut umfasst erfindungsgemäß mindestens ein Material enthaltend mindestens eine polymere Verbindung mit mindestens einer Polyurethan-Struktureinheit der Formel (I), 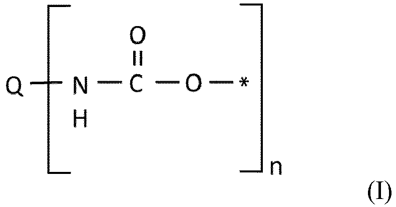 worin
Q für einen Kohlenwasserstoffrest mit 8 bis 70 Kohlenstoffatomen, bevorzugt 10 bis 30 Kohlenstoffatomen, und
n für eine Zahl von 2 bis 10, bevorzugt von 2 bis 6, besonders bevorzugt von 2 bis 4, steht und -* eine kovalente Bindung zum Polymerrückgrat bedeutet.

Q leitet sich bevorzugt ab von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohlenwasserstoffeinheiten.

Das erfindungsgemäße Verfahren eignet sich besonders gut für solche Materialien, die eine besagte polymere Verbindung enthalten, welche gemäß Formel (I) eine Gruppe Q mit mindestens einem aromatischen Rest aufweist. Als ganz besonders bevorzugte Struktureinheit der Formel (I) gelten solche, bei denen die Gruppe Q mindestens zwei alkylenverbrückte aromatische Reste, bevorzugt mindestens zwei alkylenverbrückte Phenylreste, insbesondere mindestens zwei methylenverbrückte Phenylreste, enthält.

Eine ganz besonders bevorzugte Ausführungsform des erfindungsgemäßen Verfahrens ist dadurch gekennzeichnet, dass die polymere Verbindung als mindestens eine Struktureinheit der vorgenannten Formel (I) mindestens eine Polyurethan-Struktureinheit der Formel (Ia) enthält 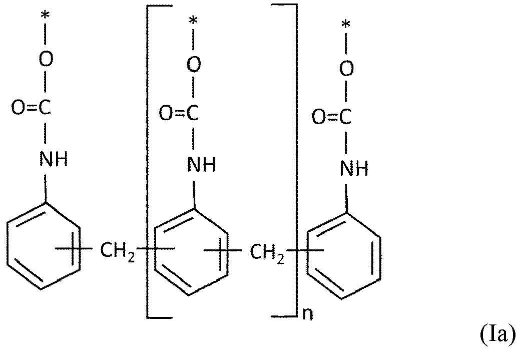 worin
n für eine Zahl von 0 bis 8, insbesondere von 0 bis 4, weiter bevorzugt von 0 bis 2, steht und - * für eine kovalente Bindung zum Polymerrückgrat steht.

Die in dem besagten Material enthaltene polymere Verbindung kann bevorzugt durch Umsetzung zumindest von
i1) mindestens einer organischen Isocyanatverbindung, enthaltend mindestens zwei bis zehn, insbesondere zwei bis acht, weiter bevorzugt zwei bis vier, Isocyanatgruppen bindend an eine Kohlenwasserstoffeinheit mit 8 bis 70 Kohlenstoffatomen, bevorzugt mit 10 bis 30 Kohlenstoffatomen; mit
i2) mindestens einer organischen Verbindung mit mindestens zwei Hydroxygruppen,
erhalten werden.

Dabei ist es im Rahmen einer weiteren Ausführungsform bevorzugt, wenn die mindestens eine organische Isocyanatverbindung als besagte Kohlenwasserstoffeinheit eine Einheit enthält, die die in i1) genannte Anzahl an Kohlenstoffatomen aufweist und sich ableitet von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohlenwas serstoffeinheiten.

Als besagte organische Isocyanatverbindung wird besonders bevorzugt mindestens eine Verbindung gemäß Formel (II) ausgewählt

Q(NCO)ₙ (II)

worin n für eine Zahl von 2 bis 4, vorzugsweise von 2 bis 3, steht und Q für einen Rest steht, ausgewählt aus einem aliphatischen Kohlenwasserstoffrest mit 8 bis 70 Kohlenstoffatomen, bevorzugt mit 10 bis 30 Kohlenstoffatomen, einem cycloaliphatischen Kohlenwasserstoffrest mit 8 bis 70 Kohlenstoffatomen, bevorzugt mit 10 bis 30 Kohlenstoffatomen, einem aromatischen Kohlenwasserstoffrest mit 8 bis 70 Kohlenstoffatomen, bevorzugt mit 10 bis 30 Kohlenstoffatomen oder einem araliphatischen Kohlenwasserstoffrest mit 8 bis 70 Kohlenstoffatomen, bevorzugt mit 10 bis 30 Kohlenstoffatomen.

Enthält besagte polymere Verbindung mindestens eine Struktureinheit der vorgenannten Formel (Ia) Ausführungsform hat es sich als weiter bevorzugte Ausführungsform des Verfahrens erwiesen, wenn in Schritt i1) als mindestens eine organische Polyisocyanatverbindung mindestens ein Polyphenylpolymethylen-polyisocyanat der Formel (III) ausgewählt wird, 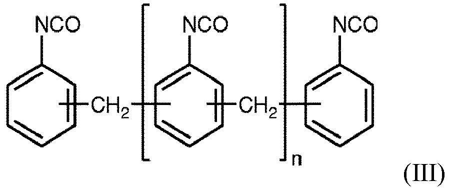 worin n für eine Zahl von 0 bis 8, insbesondere von 0 bis 4, weiter bevorzugt von 0 bis 2, steht.

Als bevorzugte Ausführungsform des vorgenannten Schrittes i2) wird mindestens eine organische Verbindung mit mindestens zwei Hydroxygruppen ausgewählt aus Polyesterpolyol, Polyetherpolyol, Polycarbonatpolyol, Polyetheresterpolyol, Polyacrylatpolyol Polyesterpolyacrylatpolyol oder Mischungen daraus.

Vorzugsweise wird mindestens eine organische Verbindung mit mindestens zwei Hydroxygruppen ausgewählt aus der Gruppe der Polyetherpolyole und/oder der Polyesterpolyole.

Die OH-Zahl des eingesetzten Polyols oder der eingesetzten Polyole kann beispielsweise > 100 mg KOH/g bis < 800 mg KOH/g betragen und die durchschnittliche OH-Funktionalität des eingesetzten Polyols oder der der eingesetzten Polyole ist > 2. Die OH-Zahl gibt im Falle eines einzelnen zugesetzten Polyols dessen OH-Zahl an. Im Falle von Mischungen wird die mittlere OH-Zahl angegeben. Dieser Wert kann anhand von DIN 53240 bestimmt werden. Die durchschnittliche OH-Funktionalität der Polyole liegt beispielsweise in einem Bereich von > 2 bis < 6.

Verwendbare Polyetherpolyole sind beispielsweise Polytetramethylenglykolpolyether, wie sie durch Polymerisation von Tetrahydrofuran mittels kationischer Ringöffnung erhältlich sind. Ebenfalls geeignete Polyetherpolyole sind Additionsprodukte von Styroloxid, Ethylenoxid, Propylenoxid, Butylenoxide und oder Epichlorhydrin an di- oder polyfunktionelle Startermoleküle. Meistens werden Polyetherpolyole mit Ethylenoxid oder Propylenoxid als Kettenverlängerer verwendet.

Geeignete Startermoleküle sind zum Beispiel Ethylenglykol, Diethylenglykol, Butyldiglykol, Glycerin, Diethylenglykol, Trimethylolpropan, Propylenglykol, Pentaerythrit, Sorbit, Saccharose, Ethylendiamin, Toluoldiamin, Triethanolamin, 1 ,4-Butandiol, 1,6-Hexandiol sowie niedermolekulare, Hydroxylgruppen aufweisende Ester derartiger Polyole mit Dicarbonsäuren.

Verwendbare Polyesterpolyole sind unter Anderem Polykondensate aus Di- sowie weiterhin Tri-, und Tetraolen und Di- sowie weiterhin Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden. Beispiele für geeignete Diole sind Ethylenglykol, Butylenglykol, Diethylenglykol, Triethylenglykol, Polyalkylenglykole wie Polyethylenglykol, weiterhin 1 ,2-Propandiol, 1,3- Propandiol, 1,3-Butandiol, 1,3-Butandiol, 1,6-Hexandiol und Isomere, Neopentylglykol oder Hydroxypivalinsäureneopentylglykolester. Daneben können auch Polyole wie Trimethylolpropan, Glycerin, Erythrit, Pentaerythrit, Trimethylolbenzol oder Trishydroxyethylisocyanurat eingesetzt werden.

Als Polycarbonsäuren können beispielsweise Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, Bernsteinsäure, 2-Methylbernsteinsäure, 3,3-Diethylglutarsäure, 2,2-Dimethylbernsteinsäure, Dodekandisäure, Endomethylentetrahydrophthalsäure, Dimerfettsäure, Trimerfettsäure, Zitronensäure oder Trimellithsäure eingesetzt werden. Als Säurequelle können auch die entsprechenden Anhydride verwendet werden.

Sofern die mittlere Funktionalität des zu veresternden Polyols > 2 ist, können zusätzlich auch Monocarbonsäuren wie Benzoesäure und Hexancarbonsäure mit verwendet werden.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind unter anderem Caprolacton, Butyrolacton und Homologe.Verwendbare Polycarbonatpolyole sind Hydroxylgruppen aufweisende Polycarbonate, zum Beispiel Polycarbonatdiole. Diese sind durch Reaktion von Kohlensäurederivaten, wie Diphenylcarbonat, Dimethylcarbonat oder Phosgen, mit Polyolen, bevorzugt Diolen oder aus Kohlendioxid erhältlich. Beispiele derartiger Diole sind Ethylenglykol, 1,2-und 1,3-Propandiol, 1,3- und 1 ,4-Butandiol, 1,6-Hexandiol, 1,8-Octandiol, Neopentylglykol, 1,4-Bishydroxymethylcyclohexan, 2-Methyl-1,3- propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art. Statt oder zusätzlich zu reinen Polycarbonatdiolen können auch Polyether- Polycarbonatdiole eingesetzt werden.

Verwendbare Polyetheresterpolyole sind solche Verbindungen, die Ethergruppen, Estergruppen und OH-Gruppen enthalten. Organische Dicarbonsäuren mit bis zu 12 Kohlenstoffatomen sind zur Herstellung der Polyetheresterpolyole geeignet, vorzugsweise aliphatische Dicarbonsäuren mit > 4 bis < 6 Kohlenstoffatomen oder aromatische Dicarbonsäuren, die einzeln oder im Gemisch verwendet werden. Beispielhaft seien Korksäure, Azelainsäure, Decandicarbonsäure, Maleinsäure, Malonsäure, Phthalsäure, Pimelinsäure und Sebacinsäure sowie insbesondere Glutarsäure, Fumarsäure, Bernsteinsäure, Adipinsäure, Phthalsäure, Terephthalsäure und Isoterephthalsäure genannt. Als Derivate dieser Säuren können beispielsweise deren Anhydride sowie deren Ester und Halbester mit niedermolekularen, monofunktionellen Alkoholen mit > 1 bis < 4 Kohlenstoffatomen eingesetzt werden.

Bevorzugt wird als mindestens eine organische Verbindung mit mindestens zwei Hydroxygruppen bevorzugt mindestens ein aliphatisches Polyesterpolyol eingesetzt, das neben von Adipinsäure abgeleiteten Baueinheiten auch solche Baueinheiten enthält, die von Glutarsäure, Bernsteinsäure und/oder Phthalsäure, bevorzugt Glutarsäure und/oder Bernsteinsäure, abgeleitet sind.

Neben der zuvor definierten Struktureinheit der Formel (I) bzw. (Ia) kann die polymere Verbindung des Materials optional zusätzlich Isocyanurat-Struktureinheiten der Formel 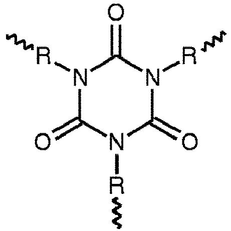 enthalten, worin R für einen bivalenten Kohlenwasserstoffrest, insbesondere für einen bivalenten aromatischen Kohlenwasserstoffrest, steht. Für diese Ausführungsform hat es sich als bevorzugt erwiesen, wenn der Anteil dieser Isocyanurat-Struktureinheiten am Gesamtgewicht des Materials höchstens 40 Gew.-% beträgt.

Im Rahmen einer Ausführungsform des erfindungsgemäßen Verfahrens, enthält das besagte Pyrolysegut das besagte Polyurethanpolymer-haltige Material in einer Gesamtmenge von 10,0 bis 80,0 Gew.-%, weiter bevorzugt 30,0 bis 70,0 Gew.-%, jeweils bezogen auf das Gesamtgewicht des Pyrolysegutes.

Bevorzugt handelt es sich bei dem die besagte polymere Verbindung enthaltenden Material um einen Schaum, noch bevorzugter um einen Polyurethanschaum. Wenn das Material in Form eines Polyurethanschaumes vorliegt, handelt es sich wiederum bevorzugt um einen Polyurethanweichschaum oder einen Polyurethanhartschaum. Ein Polyurethanhartschaum ist eine ganz besonders bevorzugte Ausführungsform des in Schritt a) des Verfahrens im Pyrolysegut eingebrachten Materials.

Wenn das besagte Material in Form eines Polyurethanhartschaumes vorliegt, hat es sich bevorzugterweise als vorteilhaft erwiesen, wenn der Polyurethanhartschaum nach DIN 7726: 1982-05 bei Druckbelastung bei 10 % Stauchung eine Druckspannung von > 15 kPa aufweist, gemessen nach DIN 53421.

Es hat sich als vorteilhaft erwiesen, wenn das besagte Material des Pyrolyseguts bevorzugt in Form festförmiger Teilchen (insbesondere in Form eines körnigen Gemenges) in den Reaktor eingebracht wird. Ein körniges Gemenge des besagten Materials wird aus einer Vielzahl an losen, festförmigen Partikeln des besagten Materials gebildet, die wiederum sogenannte Körner umfassen. Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen. Die Rieselfähigkeit eines körnigen Gemenges betrifft sein Vermögen, unter eigenem Gewicht frei aus einem Rieseltesttrichter mit einem Auslauf von 16,5 mm Durchmesser zu rieseln.

Vorzugsweise weisen die festförmigen Teilchen, insbesondere die losen, festförmigen Partikel des körnige Gemenges, des in den Reaktor eingebrachten besagten Materials einen mittleren Durchmesser X_{50,3} (Volumenmittel) von 0,01 mm bis 5 cm, bevorzugt von 0,1 mm bis 5 cm, auf. Der mittlere Teilchengrößendurchmesser X_{50,3} wird durch Siebung oder mittels eines Partikelgrößenanalysators Camsizer der Fa. Retsch bestimmt.

Die Dosierung und Prozessierbarkeit des Pyrolysegutes in der Pyrolyse des erfindungsgemäßen Verfahrens lässt sich vereinfachten, indem besagtes Pyrolysegut im Schritt (a) neben besagtem Material zusätzlich mindestens einen Füllstoff enthält. Besagter Füllstoff katalysiert bevorzugt nicht im Rahmen der Pyrolyse die thermische Zersetzung von Polyurethan. Dabei ist es wiederum besonders bevorzugt, wenn der Füllstoff mindestens ein Metalloxid ist, das im Rahmen der Pyrolyse der thermischen Zersetzung von Polyurethan nicht katalytisch wirksam ist, welches bevorzugt ausgewählt aus SiO₂.

Das besagte Material wird vorzugsweise mit einem Füllstoff z.B. Sand gemischt, wodurch eine kontinuierliche Prozessführung des erfindungsgemäßen Verfahrens vereinfacht wird. Insbesondere wird ein Verkleben des besagten Materials im Reaktor und in der Zuführung der Dosiervorrichtung zum Reaktor vermieden. Im Rahmen einer Ausführungsform wird der Füllstoff und das besagte Material im Volumenverhältnis Füllstoff zu besagtem Material von mindestens 0,1 zu 1 bis 10 zu 1 dem Pyrolysegut als Gemisch zugeführt.

Das Pyrolysegut enthält zwingend mindestens einen Katalysator. Dieser Katalysator beeinflusst die Reaktion der Zersetzung des besagten Materials. Ein entsprechender Katalysator kann die Pyrolysetemperatur herabsetzen, das Produktspektrum auf gewünschte Produkte reduzieren und gegebenenfalls die Verkokung minimieren. Für einen effizienten Prozess sind kostengünstige Katalysatoren bevorzugt.

Als effektive Katalysatoren können natürlich vorkommende Materialien wie anorganische Salze, feuerfeste Oxide, Mineralien und Industriesteine, in die optional Ionen in einem einfachen Ionenaustauschprozess ausgetauscht werden können, fungieren, da sie keine umfangreiche Synthese erfordern. Sie sind leicht verfügbar und sind daher relativ billig. Synthetische Katalysatoren wie Zeolithe (ZSM-5, ZeolithX,Y, etc) sind dagegen ein Beispiel für Katalysatoren, die zwar effektiv, aber nicht billig sind, da sie speziell hergestellt werden müssen.

Neben der Funktion als Katalysator können Katalysatoren ebenfalls die Dosierung und Prozessierbarkeit des Pyrolysegutes in der Pyrolyse vereinfachen. Bei Einsatz eines Katalysators kann in diesem Fall die eingesetzte Menge an Füllstoff reduziert werden. Im Rahmen einer Ausführungsform wird die Gesamtmenge an Füllstoff und Katalysator im Verhältnis zur Menge des besagten Materials im Volumenverhältnis von mindestens 0,1 zu 1 bis 10 zu 1 dem Pyrolysegut als Gemisch zugeführt.

Als besonders bevorzugt geeigneter, besagter Katalysator kann das Pyrolysegut mindestens einen basischen Katalysator enthalten. Handelt es sich dabei um einen festförmigen, basischen Katalysator, kann die Anzahl und Stärke der basischen Zentren des Katalysators mittels Fourier transform Infrarotspektroskopie und Temperature Programmed Desorption von CO₂ (TPD-CO2), oder mittels gängiger titrimetrischer Methoden bestimmt werden.

Bei der Pyrolysereaktion wird vorzugsweise mindestens ein Katalysator ausgewählt aus der Gruppe, die gebildet wird aus alkalischen anorganischen Materialien, bevorzugter aus der oben definierten Gruppe von natürlich vorkommenden Materialien. Diese anorganischen Materialien können feuerfeste Oxide sein. Feuerfeste Oxide sind Metalloxide, die bei hohen Temperaturen von 300°C bis 700°C stabil sind. Solche als Katalysator fungierenden Oxide umfassen die Oxide von Aluminium, Magnesium, Zirkonium, Titan, Chrom, Zink, Zinn und anderer Metalle oder Kombinationen von Aluminiumoxid mit Magnesiumoxid und/oder mit Calciumoxid.

Kristalline anorganische Materialien umfassen Aluminosilikate, SiliciumAluminiumphosphate, Silicalit, Spinelle und andere natürliche Zeolithe und Tone. Als besagter Katalysator eignet sich somit insbesondere mindestens eine Verbindung, aus der Gruppe, die gebildet wird aus anorganischen Salzen, Mineralien, Metalloxiden, Mischoxiden, Tonen, Zeolithen.

Besonders bevorzugt enthält der Katalysator mindestens ein Oxid des Aluminiums in Form eines Oxids oder Mischoxids und liegt in Spinellstruktur, Hydrotalcitstruktur oder γ-Al₂O₃-Struktur vor.

Als Katalysator eignet sich ganz besonders bevorzugt ein Mischoxid aus Al₂O₃ und MgO. Dieses wiederum liegt ganz besonders bevorzugt in Spinellstruktur oder Hydrotalcitstruktur vor.

Es hat sich z.B. gezeigt, dass wenn der Katalysator ein Mischoxid aus Al₂O₃ ist und in Hydrocalcitstruktur vorliegt, bei der Pyrolyse von MDI und/oder pMDI im Pyrolyseprodukt ein hoher Anteil an mMDA enthalten ist. Mit diesem Katalysator ließ sich somit ein höherer Anteil an zweikernigem, aromatischen Amin wiedergewinnen.

Es können sowohl homogene als auch heterogene Katalysatoren eingesetzt werden. Es sind jedoch solche Ausführungsformen des erfindungsgemäßen Verfahrens bevorzugt, in denen der Katalysator ein heterogener Katalysator ist. Es hat sich als vorteilhaft erwiesen, wenn der Katalysator bevorzugt in Form festförmiger Teilchen (insbesondere in Form eines körnigen Gemenges) in den Reaktor eingebracht wird. Der eingesetzte heterogene Katalysator weist besonders bevorzugt eine mittlere Teilchengröße seiner festförmigen Teilchen, insbesondere seiner losen, festförmigen Partikel des körnige Gemenges, einen mittleren Durchmesser X_{50,3} (Volumenmittel) von 0,01 mm bis 5 cm, bevorzugt von 0,1 mm bis 5 cm, auf. Der mittlere Teilchengrößendurchmesser X_{50,3} wird durch Siebung oder mittels eines Partikelgrößenanalysators Camsizer der Fa. Retsch bestimmt.

Bei Einsatz eines heterogenen Katalysators hat es sich im Rahmen einer weiteren bevorzugten Ausführungsform als besonders geeignet erwiesen, wenn die Katalysatorpartikel eine kleinere oder die gleiche Partikelgröße besitzen, wie die Partikel des besagten Materials. Daher ist es bevorzugt, wenn im Pyrolysegut die mittlere Teilchengröße des darin befindlichen Katalysators höchstens der mittleren Teilchengröße des darin befindlichen besagten Materials entspricht.

Wird ein Katalysator zugesetzt, kann dieser dem Füllmaterial beigemischt, auf das Füllmaterial aufgezogen, oder als Ersatz des Füllmaterials eingesetzt werden.

Da im erfindungsgemäßen Verfahren eingesetzter Katalysator dazu neigt, Ablagerungen von Koks und anderen Kohlenstoffrückständen zu erhalten, wird bei Einsatz von Katalysator bevorzugt ein Reaktor eingesetzt, der einfach und vorzugsweise die Ausbringung und kontinuierliche Regeneration des Katalysators ermöglicht. Daher ist die Verwendung eines kontinuierlichen Rührkesselreaktors (CSTR), eines bewegliches Bettes, eines Schneckenreaktors, eines Schneckenfördererreaktors, eines Flugstromreaktors, eines Drehkegelreaktors oder eines fluidisierten Bettreaktors gegenüber der Verwendung eines Festbettreaktors bevorzugt.

Der (z.B. durch Verkokung) deaktivierte Katalysator befindet sich im Pyrolyserückstand und kann nach der Ausbringung des Pyrolyserückstandes aus dem Reaktor in einen Regenerator geleitet und nach der Regeneration dem in den Pyrolysereaktor einzubringenden Pyrolysegut des Schritts (b) zugeschlagen werden. Reaktoren, die eine kurze Kontaktzeit, ein intensives Mischen der Komponenten im Beschickungsstrom mit dem Katalysator und für eine kontinuierliche Rückführung des regenerierten Katalysators in die Pyrolysezone erlauben, sind am meisten bevorzugt. Da dies bei einem Schneckenreaktor, Drehrohrofen oder Wirbelbett der Fall ist, sind diese Reaktoren besonders bevorzugt.

Das in Schritt (a) in den Reaktor eingebrachte Pyrolysegut wird gemäß Schritt (b) unter Bildung von Pyrolysat und Pyrolyserückstand zumindest teilweise zersetzt.

Das Pyrolysegut wird nach dessen Einbringung in den Reaktor auf eine Temperatur im Bereich von 250°C bis 700°C aufgeheizt. Eine besonders erfolgreiche Verbesserung des Ergebnisses des erfindungsgemäßen Verfahrens lässt sich erreichen, wenn im Rahmen einer Ausführungsform das eingebrachte Pyrolysegut auf 250°C bis 700°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes 1 Sekunde bis 2 Stunden, bevorzugt zwischen 2 Minuten und 60 Minuten, beträgt, und wobei die Temperatur und der Gehalt von Sauerstoffgas im Reaktor während dieses Zeitraums die in Schritt (b) definierten Werte betragen.

Enthält in dem erfindungsgemäßen Verfahren das Pyrolysegut einen Katalysator, befindet sich dieser Katalysator im ausgebrachten Pyrolyserückstand. Im Rahmen einer weiteren Ausführungsform des Verfahrens, wird daher der ausgebrachte Pyrolyserückstand wie oben beschrieben einem Regenerationsschritt des darin enthaltenen Katalysators zugeführt.

Davon unabhängig lassen sich gute Ergebnisse erzielen, wenn die in Schritt (b) stattfindende Herausführung des Pyrolysates aus dem Reaktor durch einen durch den Reaktor geführten Gasstrom oder durch Sog gewährleistet wird und weiter bevorzugt dadurch eine Verweildauer des Pyrolysates als Zeitraum zwischen dem Zeitpunkt der Einbringung des besagten im Schritt (a) in den Reaktor eingebrachten Materials und dem Zeitpunkt der Ausbringung des Pyrolysates 0,1 Sekunden bis 600 Sekunden, bevorzugt zwischen 0,5 Sekunden und 300 Sekunden, besonders bevorzugt 0,5 Sekunden bis 200 Sekunden, beträgt.

Wird ein Gasstrom zu diesem Zweck durch den Reaktor geführt, eignet sich als Gas für diesen Gasstrom insbesondere ein Inertgas, bevorzugt ausgewählt aus Stickstoff, Argon, CO₂, NO oder einem Gemisch daraus.

Wird ein Gasstrom zur Ausbringung des Pyrolysates genutzt, ist es erfindungsgemäß bevorzugt, wenn die Fließgeschwindigkeit des Gasstromes im Reaktor als Leerrohrgeschwindigkeit im Bereich von 0,01 m/s bis 20 m/s liegt. Sollte als Reaktor ein Festbettreaktor gewählt werden, ist es erfindungsgemäß bevorzugt, wenn die Fließgeschwindigkeit des Gasstromes im Reaktor als Leerrohrgeschwindigkeit im Bereich von 0,03 m/s bis 1 m/s liegt. Sollte als Reaktor ein Wirbelbettreaktor gewählt werden, ist es erfindungsgemäß bevorzugt, die Fließgeschwindigkeit des Gasstromes im Reaktor als Leerrohrgeschwindigkeit im Bereich von 0,5 m/s bis 2 m/s liegt. Sollte als Reaktor ein Flugstromreaktor gewählt werden, ist es erfindungsgemäß bevorzugt, die Fließgeschwindigkeit des Gasstromes im Reaktor als Leerrohrgeschwindigkeit im Bereich von 5 m/s bis 20 m/s liegt.

Als erfindungsgemäße Bedingungen für die Zersetzung im Rahmen der Pyrolyse in Schritt (b) beträgt die Temperatur im Reaktor 250 bis 700 °C und die Menge an Sauerstoffgas im Reaktor beträgt von 0 bis 2,0 Vol.% bezogen auf das Gesamtvolumen der im Reaktor befindlichen Gase.

Für die Einstellung der erfindungsgemäßen Menge an Sauerstoffgas, wird der mit besagtem Material des besagten Pyrolysegutes befüllte Reaktor mit Inertgas, insbesondere mit Stickstoff, Argon, CO₂, NO oder einer Mischung daraus befüllt. Dem Inertgas können zusätzlich von Sauerstoffgas verschiedene reaktive Gase beigemischt werden, insbesondere ausgewählt aus Methan, gasförmigem H₂O, Wasserstoffgas oder Gemischen daraus.

Um durch die Einbringung des Pyrolysegutes in den Reaktor das Eintragen von Sauerstoffgas zu minimieren, kann das Pyrolysegut vor dessen Einbringung in Schritt (a) von Sauerstoffgas befreit werden, z.B. durch Austreiben des Sauerstoffgases mittels einer Strippung mit einem Strippgas, z.B. in einem dem Reaktor vorgeschalteten Vorlagebehälter. Beispielsweise könnte als Strippgas Inertgas, insbesondere Stickstoff, Argon, CO₂, NO, Mischungen daraus, von oben oder von unten im Vorlagebehälter (bevorzugt von oben) über eine Fritte in den Behälter dem Pyrolysegut zu geführt werden, um so das Sauerstoffgas auszutreiben.

Im Rahmen einer weiteren bevorzugten Ausführungsform des Verfahrens beträgt der absolute Druck in Schritt (b) höchstens 1,2 bar.

Im Rahmen einer bevorzugten Ausführungsform des erfindungsgemäßen Verfahrens beträgt die Temperatur im Schritt (b) von 300°C bis 700°C, bevorzugt von 400°C bis 600°C.

Im Rahmen einer weiteren bevorzugten Ausführungsform des erfindungsgemäßen Verfahrens beträgt im Schritt (b) die Menge an Sauerstoffgas im Reaktor höchstens 0,5 Vol.%, bevorzugt höchstens 0,1 Vol.%, jeweils bezogen auf das Gesamtvolumen der im Reaktor befindlichen Gase.

Im Rahmen einer ganz bevorzugten Ausführungsform des erfindungsgemäßen Verfahrens beträgt im Schritt (b) erstens die Temperatur von 300°C bis 700°C, weiter bevorzugt von 400°C bis 600°C, und beträgt zweitens die Menge an Sauerstoffgas im Reaktor höchstens 0,5 Vol.%, bevorzugt höchstens 0,1 Vol.%, jeweils bezogen auf das Gesamtvolumen der im Reaktor befindlichen Gase. Dabei ist es wiederum äußerst bevorzugt, wenn zusätzlich drittens der absolute Druck in Schritt (b) höchstens 1,2 bar beträgt.

Eine bevorzugte Ausführungsform des Verfahrens liefert eine kontinuierliche Prozessführung. Dafür laufen zumindest die Schritte (a) und (b) im Rahmen einer kontinuierlichen Prozessführung gleichzeitig ab.

Das gemäß Schritt (c) erhaltene Pyrolyseprodukt kann mit gängigen Trennungsmethoden, beispielsweise Destillation oder selektive Kondensation, aufgearbeitet werden und dadurch (i) erhalten werden.

Das erfindungsgemäße Verfahren kann mit Hilfe einer entsprechend ausgestalteten Pyrolysevorrichtung durchgeführt werden. Ein weiterer Gegenstand der Erfindung ist daher die Verwendung einer Pyrolysevorrichtung enthaltend mindestens eine Dosiervorrichtung zur Einspeisung von Pyrolysegut, mindestens einen heizbaren Reaktor für die Pyrolyse, und mindestens einen Pyrolysat-Kollektor, wobei
besagter heizbarer Reaktor für die Pyrolyse mindestens ein Heizelement enthält, das zur Temperierung des Reaktors auf eine Temperatur von 250°C bis 700°C verwendet werden kann, und mindestens einen Einlass für Pyrolysegut enthält und mindestens einen davon verschiedenen Auslass für Pyrolysat enthält; und
Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
mindestens ein Pyrolysat-Kollektor mindestens eine auf eine Temperatur unter 250°C temperierte Kühlvorrichtung enthält, die im besagten Kollektor zur Senkung der Temperatur des aus dem besagten Reaktor herausgeführten Pyrolysates auf weniger als 250°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält; und
mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können,
zur Gewinnung von organischer Verbindung mit mindestens einer Aminogruppe durch Pyrolyse von polymerer Verbindung mit mindestens einer Polyurethan-Struktureinheit der Formel (I), 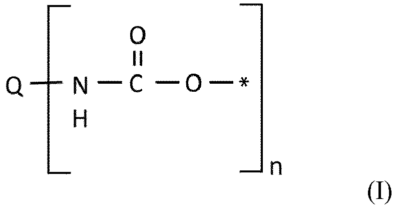 worin
Q für einen Kohlenwasserstoffrest mit 8 bis 70 Kohlenstoffatomen und
n für eine Zahl von 2 bis 10, bevorzugt von 2 bis 6, besonders bevorzugt von 2 bis 4, steht und -* eine kovalente Bindung zum Polymerrückgrat bedeutet.

Als bevorzugte polymere Verbindungen für die Pyrolyse gelten ebenfalls die im Rahmen der Beschreibung des Verfahrens genannten polymeren Verbindungen.

Es ist erfindungsgemäß bevorzugt, die besagte Pyrolysevorrichtung zur Rückgewinnung von mindestens einer aromatischen Aminoverbindung mit mindestens einer Aminogruppe, insbesondere ausgewählt aus Anilin, Toluidin, Methylendianilin (mMDA), polymerem Methylendianilin (pMDA) oder Mischungen daraus, zu verwenden.

Als Heizung des heizbaren Reaktors der besagten Pyrolysevorrichtung kann beispielsweise ein Heizelement, z.B. Heizwendel oder Heizplatten, dienen oder eine Vorrichtung zur Beheizung eines Gasstromes und zur Einbringung des beheizten Gasstroms in den Reaktor.

Der Reaktor enthält im Rahmen einer bevorzugten Ausführungsform zusätzlich mindestens einen Anschluss zu einer Gasquelle, wobei über eine Regelung, z.B. ein Ventil, ein Gasstrom im Reaktor, bevorzugt mit einer Fließgeschwindigkeit als Leerrohrgeschwindigkeit von zwischen 0,01 m/s bis 20 m/s, durch den Reaktor in den Pyrolysat-Kollektor fließt. Sollte als Reaktor ein Festbettreaktor gewählt werden, ist es erfindungsgemäß bevorzugt, wenn die Fließgeschwindigkeit des Gasstromes im Reaktor als Leerrohrgeschwindigkeit im Bereich von 0,03 m/s bis 1 m/s liegt. Sollte als Reaktor ein Wirbelbettreaktor gewählt werden, ist es erfindungsgemäß bevorzugt, die Fließgeschwindigkeit des Gasstromes im Reaktor als Leerrohrgeschwindigkeit im Bereich von 0,5 m/s bis 2 m/s liegt. Sollte als Reaktor ein Flugstromreaktor gewählt werden, ist es erfindungsgemäß bevorzugt, die Fließgeschwindigkeit des Gasstromes im Reaktor als Leerrohrgeschwindigkeit im Bereich von 5 m/s bis 20 m/s liegt. Der Gasstrom aus der Gasquelle kann beispielsweise vor der Einbringung in den Reaktor wie zuvor beschrieben durch die Heizung beheizt werden.

Der Pyrolysat-Kollektor der Pyrolysevorrichtung enthält bevorzugt eine Kühlvorrichtung, die im besagten Kollektor zur Senkung der Temperatur des aus dem besagten Reaktor herausgeführten Pyrolysates auf weniger als 50°C (weiter bevorzugt auf weniger als 30°C) unter Bildung von Pyrolyseprodukt verwendbar ist. Hierfür eignen sich bevorzugt Kühlaggregate die nach dem Wärmetauscherprinzip arbeiten. Dabei kann der Pyrolysat-Kollektor als selektiver Kondensator für eine selektive Kondensation von im Pyrolysat enthaltenen Pyrolyseprodukt-Bestandteilen ausgerüstet sein.

Unter einer "Fluidverbindung" wird erfindungsgemäß ein Teil der Vorrichtung verstanden, der Anlagenteile miteinander verbindet und durch die sich ein Stoff, der in jedem Aggregatzustand vorliegen kann, von einem Anlagenteil zum nächsten transportieren lässt, beispielsweise eine Zuleitung in Form eines Rohres.

Das erfindungsgemäße Verfahren, sowie die erfindungsgemäße Vorrichtung tragen zur Lösung der zuvor gestellten Aufgabe bei und liefern nach Schritt (c) des Verfahrens als Pyrolyseprodukt eine Zusammensetzung, mindestens enthaltend jeweils bezogen auf das Gesamtgewicht der Zusammensetzung
(i) von 0 und 40 Gew.-% einer Gesamtmenge von mindestens einer aromatischen Verbindung mit mindestens zwei Aminogruppen,
(ii) zwischen 0 und 35 Gew.-% einer Gesamtmenge von mindestens einer aromatischen Verbindung mit genau einer Aminogruppe, bevorzugt von Anilin,
(iii) von 0 bis 40 Gew.-% einer Gesamtmenge mindestens einer Kohlenwasserstoff-Verbindung, enthaltend mindestens eine funktionelle Gruppe mit mindestens einem Sauerstoffatom und keine funktionelle Gruppe mit Stickstoffatom,
(iv) wobei die Gewichtsanteile innerhalb der Gewichtsanteilbereiche aus (i), (ii) und (iii) derart ausgewählt werden, dass die Summe der gewählten Gewichtanteile aus (i), (ii) und (iii) gemeinsam mit den Gewichtsanteilen weiterer Inhaltsstoffe 100 Gew.-% ergibt.

Im Rahmen einer bevorzugten Ausführungsform enthält die Zusammensetzung unter (i) von 0 bis 40 Gew.-% Verbindung der Formel (IV) 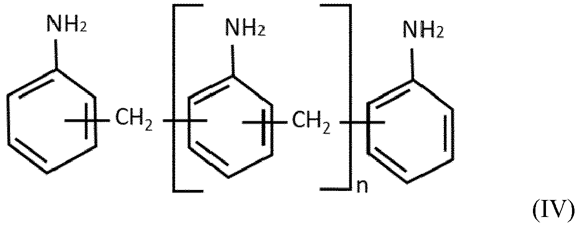 worin n für eine Zahl von 0 bis 8, insbesondere von 0 bis 4, weiter bevorzugt von 0 bis 2, steht.

Weiter bevorzugte Pyrolyseprodukte enthalten mehr (i) aromatische Verbindungen mit mindestens zwei Aminogruppen als (ii) aromatische Verbindungen mit genau einer Aminogruppe.

Im Rahmen einer bevorzugten Ausführungsform enthält die Zusammensetzung unter (ii) zwischen 0 und 35 Gew.-% einer Gesamtmenge von aromatischer Aminoverbindung mit mindestens einer Aminogruppe, ausgewählt aus Anilin, Toluidin oder Mischungen daraus.

Es ist zudem im Rahmen einer anderen bevorzugten Ausführungsform vorteilhaft, wenn das Pyrolyseprodukt zwischen 0 und 40 Gew.-% einer Gesamtmenge von mindestens einer aromatischen Verbindung mit mindestens zwei Aminogruppen enthält.

Im Rahmen einer bevorzugten Ausführungsform enthält die Zusammensetzung unter (iii) zwischen 0 und 40 Gew.-% Kohlenwasserstoff-Verbindungen, enthaltend mindestens eine funktionelle Gruppe mit mindestens einem Sauerstoffatom und keine funktionelle Gruppe mit Stickstoffatom. Diese Kohlenwasserstoffverbindungen werden wiederum besonders bevorzugt ausgewählt aus Verbindunen mit einer Molmasse von höchstens 300 g/mol, insbesondere höchstens 250 g/mol. Weiter bevorzugt werden die besagten Kohlenwasserstoffverbindungen ausgewählt aus Aceton, Dimethyldioxan, Propen, 1,2-Propylenglycol, Dipropylenglycol, Tripropylenglycol oder Tetrapropylenglycol.

Zusammenfassend aber nicht beschränkend gelten folgende Aspekte 1 bis 30 der Erfindung als weitere Ausführungsformen, deren Merkmale nach vorstehender Beschreibung der Erfindung auszulegen sind:
1. Verfahren zur Pyrolyse, umfassend mindestens die folgenden Schritte
   (a) Einbringung von Pyrolysegut, mindestens umfassend
      i) Material enthaltend mindestens eine polymere Verbindung mit mindestens einer Polyurethan-Struktureinheit der Formel (I), 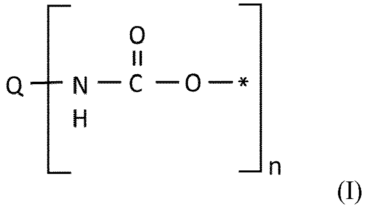 worin
         Q für einen Kohlenwasserstoffrest mit 8 bis 70 Kohlenstoffatomen, bevorzugt 10 bis 30 Kohlenstoffatomen, und
         n für eine Zahl von 2 bis 10, bevorzugt von 2 bis 6, besonders bevorzugt von 2 bis 4, steht
         und -* eine kovalente Bindung zum Polymerrückgrat bedeutet, und
      ii) mindestens einen, die thermische Zersetzung der besagten polymeren Verbindung beeinflussenden Katalysator,
      in einen Reaktor;
   (b) Zersetzung zumindest des in Schritt (a) eingebrachten Materials des Pyrolyseguts in dem Reaktor bei einer Temperatur von 250°C bis 700°C, unter Erhalt von in der Gasphase befindlichem Produkt als Pyrolysat und von nicht in der Gasphase befindlichem Pyrolyserückstand, wobei
      (i) während der besagten Zersetzung die Menge an Sauerstoffgas im Reaktor höchstens 2,0 Vol.% bezogen auf das Gesamtvolumen der im Reaktor befindlichen Gase beträgt, und
      (ii) während der besagten Zersetzung das Pyrolysat aus dem Reaktor herausgeführt wird, und
      (iii) der besagte Pyrolyserückstand aus dem Reaktor herausgeführt wird;
   (c) Abkühlung des herausgeführten Pyrolysates auf eine Temperatur von weniger als 250°C unter Erhalt von Pyrolyseprodukt, ausgewählt aus Pyrolysatkondensat, Pyrolysatsublimat oder einer Mischung daraus;
   (d) optional Aufarbeitung des Pyrolyseprodukts.
2. Verfahren nach Aspekt 1, dadurch gekennzeichnet, dass der Reaktor ausgewählt wird aus kontinuierlichem Rührkesselreaktor (CSTR), Festbettreaktor, Fließbettreaktor, Schneckenreaktor, Schneckenförderer Reaktor, Flugstromreaktor, Drehrohrreaktor, Schaufelreaktor, insbesondere ausgewählt wird aus kontinuierlichem Rührkesselreaktor (CSTR), Festbettreaktor (insbesondere mit kontinuierlichem Bettaustausch (Schachtreaktor) bevorzugt mit innenliegenden Wärmetauscherrohren), ein Schneckenreaktor, ein Schneckenfördererreaktor, ein Flugstromreaktor, Drehrohrreaktor oder Wirbelschichtreaktor.
3. Verfahren nach einem der Aspekte 1 oder 2, dadurch gekennzeichnet, dass die Temperatur im Schritt (b) von 300°C bis 700°C, bevorzugt 400°C bis 600°C beträgt.
4. Verfahren nach einem der vorangehenden Aspekte, dadurch gekennzeichnet, dass der absolute Druck in Schritt (b) höchstens 1,2 bar beträgt.
5. Verfahren nach einem der vorangehenden Aspekte, dadurch gekennzeichnet, dass das eingebrachte Pyrolysegut auf 250°C bis 700°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes 1 Sekunde bis 5 Stunden, bevorzugt zwischen 2 Minuten und 120 Minuten, beträgt.
6. Verfahren nach einem der vorangehenden Aspekte, dadurch gekennzeichnet, dass die Herausführung des Pyrolysates aus dem Reaktor durch einen durch den Reaktor geführten Gasstrom oder durch Sog gewährleistet wird und bevorzugt dadurch eine Verweildauer des Pyrolysates als Zeitraum zwischen dem Zeitpunkt der Bildung des Pyrolysats und dem Zeitpunkt der Ausbringung des resultierenden Pyrolysates aus dem Reaktor 0,1 Sekunden bis 600 Sekunden, bevorzugt zwischen 0,5 Sekunden und 300 Sekunden, besonders bevorzugt 0,5 Sekunden bis 200 Sekunden, beträgt.
7. Verfahren nach einem der vorangehenden Aspekte, dadurch gekennzeichnet, dass die Herausführung des Pyrolysates aus dem Reaktor durch einen durch den Reaktor geführten Gasstrom gewährleistet wird, dessen Fließgeschwindigkeit im Reaktor als Leerrohrgeschwindigkeit im Bereich von 0,01 m/s bis 20 m/s liegt.
8. Verfahren nach einem der vorangehenden Aspekte, dadurch gekennzeichnet, dass der Pyrolyserückstand im Reaktor festförmig ist.
9. Verfahren nach einem der vorangehenden Aspekte, dadurch gekennzeichnet, dass zumindest die Schritte (a) und (b) im Rahmen einer kontinuierlichen Prozessführung gleichzeitig ablaufen.
10. Verfahren nach einem der vorangehenden Aspekte, dadurch gekennzeichnet, dass im Schritt (b) die Menge an Sauerstoffgas im Reaktor höchstens 0,5 Vol.%, bevorzugt höchstens 0,1 Vol.%, jeweils bezogen auf das Gesamtvolumen der im Reaktor befindlichen Gase beträgt.
11. Verfahren nach einem der vorangehenden Aspekte, dadurch gekennzeichnet, dass der mit besagten Material befüllte Reaktor mit Inertgas, insbesondere mit Stickstoff, Argon, CO₂, NO oder Mischungen daraus durchströmt wird.
12. Verfahren nach Aspekt 11, dadurch gekennzeichnet, dass dem Inertgas von Sauerstoffgas verschiedene reaktive Gase beigemischt werden, insbesondere ausgewählt aus Methan, gasförmigem H₂O, Wasserstoffgas oder Gemischen daraus.
13. Verfahren nach einem der vorangehenden Aspekte, dadurch gekennzeichnet, dass die polymere Verbindung des Polyurethan-Materials zusätzlich Isocyanurat-Struktureinheiten der Formel 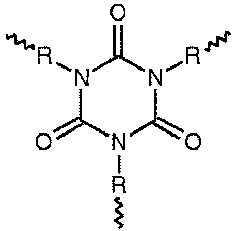 enthält, worin R für einen bivalenten Kohlenwasserstoffrest, insbesondere für einen bivalenten aromatischen Kohlenwasserstoffrest, steht.
14. Verfahren nach einem der vorangehenden Aspekte, dadurch gekennzeichnet, dass die polymere Verbindung mindestens eine Polyurethan-Struktureinheit der Formel (Ia) enthält 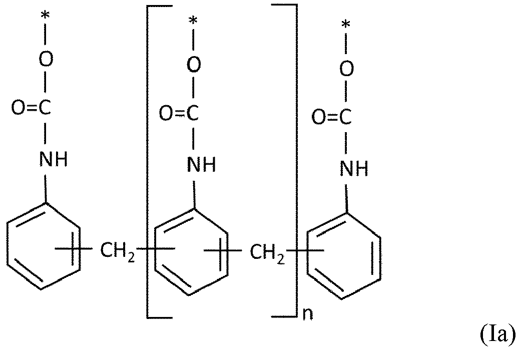 worin
   n für eine Zahl von 0 bis 8, insbesondere von 0 bis 4, weiter bevorzugt von 0 bis 2, steht und -* für eine kovalente Bindung zum Polymerrückgrat steht.
15. Verfahren nach einem der vorangehenden Aspekte, dadurch gekennzeichnet, dass die polymere Verbindung durch Umsetzung zumindest von
   i1) mindestens einer organischen Isocyanatverbindung, enthaltend mindestens zwei bis zehn, insbesondere zwei bis acht, weiter bevorzugt zwei bis vier, Isocyanatgruppen bindend an eine Kohlenwasserstoffeinheit mit 8 bis 70 Kohlenstoffatomen, bevorzugt mit 10 bis 30 Kohlenstoffatomen; mit
   i2) mindestens einer organischen Verbindung mit mindestens zwei Hydroxygruppen,
   erhältlich ist.
16. Verfahren nach Aspekt 15, dadurch gekennzeichnet, dass die mindestens eine organische Isocyanatverbindung als besagte Kohlenwasserstoffeinheit eine Einheit enthält, die die zuvor genannte Anzahl an Kohlenstoffatomen aufweist und sich ableitet von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohlenwasserstoffeinheiten.
17. Verfahren nach Aspekt 15 oder 16, dadurch gekennzeichnet, dass als besagte organische Isocyanatkomponente mindestens eine Verbindung gemäß Formel (II) ausgewählt wird

   Q(NCO)ₙ (II)

   worin n für eine Zahl von 2 bis 4, vorzugsweise von 2 bis 3, steht und Q für einen Rest steht, ausgewählt aus einem aliphatischen Kohlenwasserstoffrest mit 8 bis 70 Kohlenstoffatomen, bevorzugt mit 10 bis 30 Kohlenstoffatomen, einem cycloaliphatischen Kohlenwasserstoffrest mit 8 bis 70 Kohlenstoffatomen, bevorzugt mit 10 bis 30 Kohlenstoffatomen, einem aromatischen Kohlenwasserstoffrest mit 8 bis 70 Kohlenstoffatomen, bevorzugt mit 10 bis 30 Kohlenstoffatomen oder einem araliphatischen Kohlenwasserstoffrest mit 8 bis 70 Kohlenstoffatomen, bevorzugt mit 10 bis 30 Kohlenstoffatomen.
18. Verfahren nach einem der Aspekte 1 bis 17, dadurch gekennzeichnet, dass in der entsprechenden Formel (I) und für Aspekt 18 auch in Formel (II) Q mindestens zwei alkylenverbrückte aromatische Reste, bevorzugt mindestens zwei alkylenverbrückte Phenylreste, insbesondere zwei methylenverbrückte Phenylreste, enthält.
19. Verfahren nach einem der Aspekte 15 bis 18, dadurch gekennzeichnet, dass als organische Polyisocyanatkomponente mindestens ein Polyphenylpolymethylen-polyisocyanat der Formel (III) ausgewählt wird, 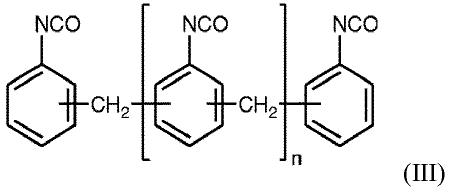 worin n für eine Zahl von 0 bis 8, insbesondere von 0 bis 4, weiter bevorzugt von 0 bis 2, steht.
20. Verfahren nach einem der Aspekte 15 bis 19, dadurch gekennzeichnet, dass mindestens eine organische Verbindung mit mindestens zwei Hydroxygruppen ausgewählt wird aus Polyesterpolyol, Polyetherpolyol, Polycarbonatpolyol, Polyetheresterpolyol, Polyacrylatpolyol Polyesterpolyacrylatpolyol oder Mischungen daraus, bevorzugt mindestens ein aliphatisches Polyesterpolyol, das neben von Adipinsäure abgeleiteten Baueinheiten auch solche Baueinheiten enthält, die von Glutarsäure, Bernsteinsäure und/oder Phthalsäure, bevorzugt Glutarsäure und/oder Bernsteinsäure, abgeleitet sind.
21. Verfahren nach einem der vorangehenden Aspekte, dadurch gekennzeichnet, dass das Material als Polyurethanhartschaum in dem Pyrolysegut vorliegt, bevorzugt als Polyurethanhartschaum, der nach DIN 7726: 1982-05 bei Druckbelastung bei 10 % Stauchung eine Druckspannung von > 15 kPa aufweist, gemessen nach DIN 53421.
22. Verfahren nach einem der vorangehenden Aspekte, dadurch gekennzeichnet, dass besagtes Material in Form festförmiger Teilchen, insbesondere in Form eines körnigen Gemenges, in den Reaktor eingebracht wird.
23. Verfahren nach Aspekt 22, dadurch gekennzeichnet, dass die festförmigen Teilchen, insbesondere die losen, festförmigen Partikel des körnige Gemenges, des in den Reaktor eingebrachten besagten Materials eine einen mittleren Durchmesser X_{50,3} (Volumenmittel) von 0,01mm bis 5 cm aufweisen.
24. Verfahren nach einem der vorangehenden Aspekte, dadurch gekennzeichnet, dass der Katalysator mindestens eine Verbindung ist, aus der Gruppe, die gebildet wird aus anorganischen Salzen, Mineralien, Metalloxiden, Mischoxiden, Tonen, Zeolithen, bevorzugt ein Mischoxid aus Al₂O₃ und MgO ist.
25. Verfahren nach einem der vorangehenden Aspekte, dadurch gekennzeichnet, dass der Katalysator ein basischer Katalysator ist.
26. Verfahren nach einem der vorangehenden Aspekte, dadurch gekennzeichnet, dass der Katalysator ein heterogener Katalysator ist.
27. Verfahren nach einem der vorangehenden Aspekte, dadurch gekennzeichnet, dass besagtes Pyrolysegut bezogen auf dessen Gesamtgewicht besagtes Material in einer Gesamtmenge von 10,0 bis 80,0 Gew.-%, weiter bevorzugt 30,0 bis 70,0 Gew.-%, enthält.
28. Verwendung einer Pyrolysevorrichtung enthaltend mindestens eine Dosiervorrichtung zur Einspeisung von Pyrolysegut, mindestens einen heizbaren Reaktor für die Pyrolyse, und mindestens einen Pyrolysat-Kollektor, wobei
   besagter heizbarer Reaktor für die Pyrolyse mindestens ein Heizelement enthält, das zur Temperierung des Reaktors auf eine Temperatur von 250°C bis 700°C verwendet werden kann, und mindestens einen Einlass für Pyrolysegut enthält und mindestens einen davon verschiedenen Auslass für Pyrolysat enthält; und
   Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
   heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
   mindestens ein Pyrolysat-Kollektor mindestens eine auf eine Temperatur unter 250°C temperierte Kühlvorrichtung enthält, die im besagten Kollektor zur Senkung der Temperatur des aus dem besagten Reaktor herausgeführten Pyrolysates auf weniger als 250°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält; und
   mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können,
   zur Gewinnung von organischer Verbindung mit mindestens einer Aminogruppe durch Pyrolyse von polymerer Verbindung mit mindestens einer Polyurethan-Struktureinheit der Formel (I), 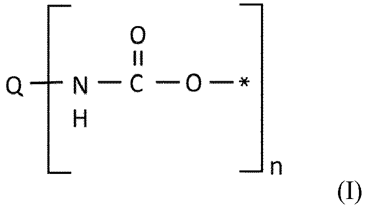 worin
   Q für einen Kohlenwasserstoffrest mit 8 bis 70 Kohlenstoffatomen und
   n für eine Zahl von 2 bis 10, bevorzugt von 2 bis 6, besonders bevorzugt von 2 bis 4, steht
   und -* eine kovalente Bindung zum Polymerrückgrat bedeutet.
29. Verwendung nach Aspekt 28, dadurch gekennzeichnet, dass die Pyrolyse-Vorrichtung zur Rückgewinnung von mindestens einer aromatischen Aminoverbindung mit mindestens einer Aminogruppe, insbesondere ausgewählt aus Anilin, Toluidin, Methylendianilin (mMDA), polymerem Methylendianilin (pMDA) oder Mischungen daraus, verwendet wird.
30. Zusammensetzung, mindestens enthaltend jeweils bezogen auf das Gesamtgewicht der Zusammensetzung
   (i) von 0 und 40 Gew.-% einer Gesamtmenge von mindestens einer aromatischen Verbindung mit mindestens zwei Aminogruppen, ,
   (ii) zwischen 0 und 35 Gew.-% einer Gesamtmenge von mindestens einer aromatischen Verbindung mit genau einer Aminogruppe, bevorzugt von Anilin,(iii) zwischen 0 und 40 Gew.-% Kohlenwasserstoff-Verbindungen, enthaltend mindestens eine funktionelle Gruppe mit mindestens einem Sauerstoffatomund keine funktionelle Gruppe mit Stickstoffatom,
   (iii) von 0 bis 40 Gew.-% einer Gesamtmenge mindestens einer Kohlenwasserstoff-Verbindung, enthaltend mindestens eine funktionelle Gruppe mit mindestens einem Sauerstoffatom und keine funktionelle Gruppe mit Stickstoffatom,
   (iv) wobei die Gewichtsanteile innerhalb der Gewichtsanteilbereiche aus (i), (ii) und (iii) derart ausgewählt werden, dass die Summe der gewählten Gewichtanteile aus (i), (ii) und (iii) gemeinsam mit den Gewichtsanteilen weiterer Inhaltsstoffe 100 Gew.-% ergibt.

### Beispiele

### a) Bereitstellung des Pyrolysegutes:

Es wurde ein Polyurethanhartschaum mittels Standardverfahren aus den Komponenten gemäß Tabelle 1 hergestellt.

**Tabelle 1: Komponenten des Hartschaumes:**

| **Bestandteile** | **Gew.-%** |
|---|---|
| Polyol mit einer OH Zahl von 450 | 30,10 |
| Polyol mit o-Toluylendiamin als Starter mit einer OH-Zahl von 400 | 8,60 |
| Polyol mit einer OH Zahl 112 | 2,58 |
| Mischung aus Polycat^{®} 5, Polycat^{®} 8 und Polycat^{®} 41 ¹ | 0,86 |
| Silikon (Stabilisator) | 0,86 |

| **Isocyanat** | |
|---|---|
| Desmodur^{®} 44V20 (pMDI) | 57,00 |

| | |
|---|---|
| ¹ Katalysatoren, vertrieben von der Firma Evonik, Deutschland ² flüssiges, dunkelbraunes Isocyanat der Firma Covestro Deutschland AG auf Basis von Diphenylmethan-4,4'-diisocyanat (MDI) mit Isomeren und höherfunktionellen Homologen (pMDI) | |

Zur Bereitstellung von verschiedenem Pyrolysegut P1 bis P3 wurden 1 g des klein geraspelten Polyurethan Hartschaums mit 4 g eines in der Tabelle 2 verzeichneten Katalysatorpulvers innig vermischt. Pyrolysegut P4 enthielt zum Vergleich keinen Katalysator.

**Tabelle 2: Katalysator K1 bis K3 und Pyrolysegut P1 bis P3**

| **Katalysator:** | **K1** | **K2** | **K3** |
|---|---|---|---|
| im Pyrolysegut: | **P1** | **P2** | **P3** |
| Gew% MgO | 5,0 | 70,0 | 0,0 |
| Gew.% Al₂O3 | 95,0 | 30,0 | 100,0 |
| Struktur | basischer Al₂O₃ precursor | Hydrotalcit | γ-Al₂O₃ |
| Korngröße dso [µm] | 40 | 40 | 40 |

### b) Durchführung der Pyrolyse:

Die Pyrolyse des Polyurethan Hartschaums , wurde in einem mit N₂ durchströmten Festbettreaktor mit einem Volumen von 25 ml bei 500 °C durchgeführt.

Die Fließgeschwindigkeit des Stickstoff Gasstroms (Leerrohrgeschwindigkeit) im Reaktor betrug 0,07 m/s. Ein Pyrolysegut gemäß Tabelle 1 wurde in den Reaktor eingebracht. Die Verweilzeit des eingebrachten Polyurethan-Materials betrugt 30 min. Der Reaktor wurde auf 500 °C auf geheizt und dann 30 min bei dieser Temperatur gehalten. Hinter dem Reaktor befanden sich 3 Kondensatoren, um die Flüssigkomponenten aus dem entstehenden Pyrolysegas abzutrennen. Der entstehende Koksanteil wurde durch Wiegen des Katalysatorpulvers nach der Pyrolyse bestimmt. Das Gas nach den 3 Kondensatoren wurde mittels online IR charakterisiert. Die Komponenten im als Öl anfallendem Pyrolyseprodukt wurden mit GC-FID bestimmt. Dazu wurde ein Agilent 7890A mit einer Säule SupelcoSPB 50 benutzt. Das Pyrolyse Öl wurde mit Aceton 1:50 oder 1:100 verdünnt.

**Tabelle 3: Zusammensetzung des erhaltenen Pyrolysats [Gew.-%]:**

| **Pyrolysegut** | **P1** | **P2** | **P3** | **P4** |
|---|---|---|---|---|
| Katalysator | K1 | K2 | K3 | ohne |
| Anilin* | 19,0 | 11,0 | 10,0 | - |
| Methylanilin* | 8,3 | 3,0 | 5,0 | - |
| mMDA* | 2,3 | 20,0 | - | 4,1 |
| Öl (nicht charakterisiert) enthaltend pMDA* | 40,0 | 29,0 | 35,0 | 53,2 |
| | | | | |

| Gasphase nach Kondensation | | | | |
|---|---|---|---|---|
| H₂O | - | 0,5 | 20,0 | - |
| CO₂ | 10,0 | 13,0 | - | 10,0 |
| CO | - | 3,0 | - | - |
| Koks | 10,0 | 10,0 | 30,0 | 17,3 |
| weitere Gase | 10,4 | 5,5 | - | 15,7 |
| | | | | |
| Gesamt | 100,0 | 100,0 | 100,0 | 100,0 |

| | | | | |
|---|---|---|---|---|
| * kennzeichnet Bestandteile des Pyrolyseproduktes | | | | |

Mit dem Katalysator K2 enthaltendem Pyrolysegut P2 wurde jeweils ein Pyrolyseexperiment bei 500°C und ein Pyrolyseexperiment bei 470°C durchgeführt. Die Verteilung der erhaltenen Amine wurde verglichen (siehe Tabelle 4). Dabei wurde festgestellt, dass bei 470°C mehr Methylendianilin (mMDA) im Pyrolyseprodukt erhalten wird.

**Tabelle 4: Vergleich der Verteilung der erhaltenen aromatischen Amine [Gew.%]:**

| | **P2** | **P2** |
|---|---|---|
| **Produkt** | **500 °C** | **470°C** |
| Aniline | 11 | 3 |
| Methylaniline | 3 | 3 |
| mMDA | 20 | 30 |

## Patentansprüche

1. Verfahren zur Pyrolyse, umfassend mindestens die folgenden Schritte
(a) Einbringung von Pyrolysegut, mindestens umfassend
i) Material enthaltend mindestens eine polymere Verbindung mit mindestens einer Polyurethan-Struktureinheit der Formel (I), 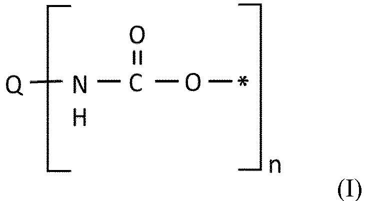 worin
Q für einen Kohlenwasserstoffrest mit 8 bis 70 Kohlenstoffatomen, bevorzugt 10 bis 30 Kohlenstoffatomen, und
n für eine Zahl von 2 bis 10, bevorzugt von 2 bis 6, besonders bevorzugt von 2 bis 4, steht
und -* eine kovalente Bindung zum Polymerrückgrat bedeutet, und
ii) mindestens einen, die thermische Zersetzung der besagten polymeren Verbindung beeinflussenden Katalysator,
in einen Reaktor;
(b) Zersetzung zumindest des in Schritt (a) eingebrachten Materials des Pyrolyseguts in dem Reaktor bei einer Temperatur von 250°C bis 700°C, unter Erhalt von in der Gasphase befindlichem Produkt als Pyrolysat und von nicht in der Gasphase befindlichem Pyrolyserückstand, wobei
(i) während der besagten Zersetzung die Menge an Sauerstoffgas im Reaktor höchstens 2,0 Vol.% bezogen auf das Gesamtvolumen der im Reaktor befindlichen Gase beträgt, und
(ii) während der besagten Zersetzung das Pyrolysat aus dem Reaktor herausgeführt wird, und
(iii) der besagte Pyrolyserückstand aus dem Reaktor herausgeführt wird;
(c) Abkühlung des herausgeführten Pyrolysates auf eine Temperatur von weniger als 250°C unter Erhalt von Pyrolyseprodukt, ausgewählt aus Pyrolysatkondensat, Pyrolysatsublimat oder einer Mischung daraus;
(d) optional Aufarbeitung des Pyrolyseprodukts.

2. Verfahren nach einem der Ansprüche 1 oder 2, **dadurch gekennzeichnet, dass** die Temperatur im Schritt (b) von 300°C bis 700°C, bevorzugt 400°C bis 600°C beträgt.

3. Verfahren nach einem der vorangehenden Ansprüche, **dadurch gekennzeichnet, dass** der absolute Druck in Schritt (b) höchstens 1,2 bar beträgt.

4. Verfahren nach einem der vorangehenden Ansprüche, **dadurch gekennzeichnet, dass** das eingebrachte Pyrolysegut auf 250°C bis 700°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes 1 Sekunde bis 5 Stunden, bevorzugt zwischen 2 Minuten und 120 Minuten, beträgt.

5. Verfahren nach einem der vorangehenden Ansprüche, **dadurch gekennzeichnet, dass** die Herausführung des Pyrolysates aus dem Reaktor durch einen durch den Reaktor geführten Gasstrom oder durch Sog gewährleistet wird und bevorzugt dadurch eine Verweildauer des Pyrolysates als Zeitraum zwischen dem Zeitpunkt der Bildung des Pyrolysats und dem Zeitpunkt der Ausbringung des resultierenden Pyrolysates aus dem Reaktor 0,1 Sekunden bis 600 Sekunden, bevorzugt zwischen 0,5 Sekunden und 300 Sekunden, besonders bevorzugt 0,5 Sekunden bis 200 Sekunden, beträgt.

6. Verfahren nach einem der vorangehenden Ansprüche, **dadurch gekennzeichnet, dass** die Herausführung des Pyrolysates aus dem Reaktor durch einen durch den Reaktor geführten Gasstrom gewährleistet wird, dessen Fließgeschwindigkeit im Reaktor als Leerrohrgeschwindigkeit im Bereich von 0,01 m/s bis 20 m/s liegt.

7. Verfahren nach einem der vorangehenden Ansprüche, **dadurch gekennzeichnet, dass** zumindest die Schritte (a) und (b) im Rahmen einer kontinuierlichen Prozessführung gleichzeitig ablaufen.

8. Verfahren nach einem der vorangehenden Ansprüche, **dadurch gekennzeichnet, dass** im Schritt (b) die Menge an Sauerstoffgas im Reaktor höchstens 0,5 Vol.%, bevorzugt höchstens 0,1 Vol.%, jeweils bezogen auf das Gesamtvolumen der im Reaktor befindlichen Gase beträgt.

9. Verfahren nach einem der vorangehenden Ansprüche, **dadurch gekennzeichnet, dass** der mit besagten Material befüllte Reaktor mit Inertgas, insbesondere mit Stickstoff, Argon, CO₂, NO oder Mischungen daraus durchströmt wird.

10. Verfahren nach einem der vorangehenden Ansprüche, **dadurch gekennzeichnet, dass** die polymere Verbindung mindestens eine Polyurethan-Struktureinheit der Formel (Ia) enthält 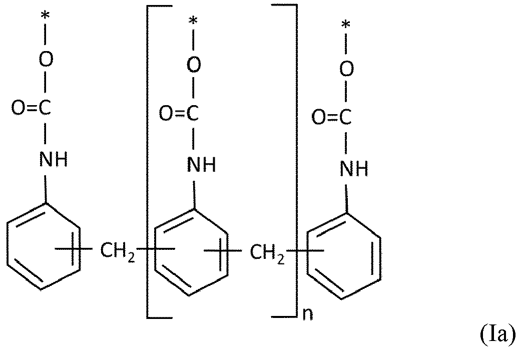 worin
n für eine Zahl von 0 bis 8, insbesondere von 0 bis 4, weiter bevorzugt von 0 bis 2, steht und -* für eine kovalente Bindung zum Polymerrückgrat steht.

11. Verfahren nach einem der vorangehenden Ansprüche, **dadurch gekennzeichnet, dass** die polymere Verbindung durch Umsetzung zumindest von
i1) mindestens einer organischen Isocyanatverbindung, enthaltend mindestens zwei bis zehn, insbesondere zwei bis acht, weiter bevorzugt zwei bis vier, Isocyanatgruppen bindend an eine Kohlenwasserstoffeinheit mit 8 bis 70 Kohlenstoffatomen, bevorzugt mit 10 bis 30 Kohlenstoffatomen; mit
i2) mindestens einer organischen Verbindung mit mindestens zwei Hydroxygruppen,
erhältlich ist.

12. Verfahren nach Anspruch 15, **dadurch gekennzeichnet, dass** die mindestens eine organische Isocyanatverbindung als besagte Kohlenwasserstoffeinheit eine Einheit enthält, die die zuvor genannte Anzahl an Kohlenstoffatomen aufweist und sich ableitet von aliphatischen Kohlenwasserstoffeinheiten, cycloaliphatischen Kohlenwasserstoffeinheiten, araliphatischen Kohlenwasserstoffeinheiten, aromatischen Kohlenwasserstoffeinheiten oder heterozyklischen Kohlenwasserstoffeinheiten.

13. Verfahren nach einem der Ansprüche 15 bis 17, **dadurch gekennzeichnet, dass** als organische Polyisocyanatkomponente mindestens ein Polyphenylpolymethylen-polyisocyanat der Formel (III) ausgewählt wird, 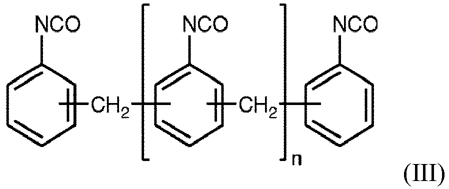 worin n für eine Zahl von 0 bis 8, insbesondere von 0 bis 4, weiter bevorzugt von 0 bis 2, steht.

14. Verfahren nach einem der vorangehenden Ansprüche, **dadurch gekennzeichnet, dass** besagtes Material in Form festförmiger Teilchen, insbesondere in Form eines körnigen Gemenges, in den Reaktor eingebracht wird.

15. Verfahren nach einem der vorangehenden Ansprüche, **dadurch gekennzeichnet, dass** der Katalysator mindestens eine Verbindung ist, aus der Gruppe, die gebildet wird aus anorganischen Salzen, Mineralien, Metalloxiden, Mischoxiden, Tonen, Zeolithen, bevorzugt ein Mischoxid aus Al₂O₃ und MgO ist.

16. Verfahren nach einem der vorangehenden Ansprüche, **dadurch gekennzeichnet, dass** der Katalysator ein basischer Katalysator ist.

17. Verfahren nach einem der vorangehenden Ansprüche, **dadurch gekennzeichnet, dass** der Katalysator ein heterogener Katalysator ist.

18. Verwendung einer Pyrolysevorrichtung enthaltend mindestens eine Dosiervorrichtung zur Einspeisung von Pyrolysegut, mindestens einen heizbaren Reaktor für die Pyrolyse, und mindestens einen Pyrolysat-Kollektor, wobei
besagter heizbarer Reaktor für die Pyrolyse mindestens ein Heizelement enthält, das zur Temperierung des Reaktors auf eine Temperatur von 250°C bis 700°C verwendet werden kann, und mindestens einen Einlass für Pyrolysegut enthält und mindestens einen davon verschiedenen Auslass für Pyrolysat enthält; und
Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
mindestens ein Pyrolysat-Kollektor mindestens eine auf eine Temperatur unter 250°C temperierte Kühlvorrichtung enthält, die im besagten Kollektor zur Senkung der Temperatur des aus dem besagten Reaktor herausgeführten Pyrolysates auf weniger als 250°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält; und
mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können,
zur Gewinnung von organischer Verbindung mit mindestens einer Aminogruppe durch Pyrolyse von polymerer Verbindung mit mindestens einer Polyurethan-Struktureinheit der Formel (I), 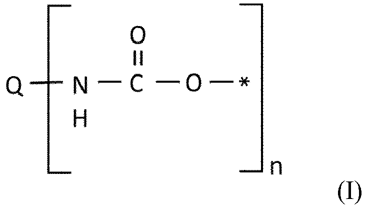 worin
Q für einen Kohlenwasserstoffrest mit 8 bis 70 Kohlenstoffatomen und
n für eine Zahl von 2 bis 10, bevorzugt von 2 bis 6, besonders bevorzugt von 2 bis 4, steht
und -* eine kovalente Bindung zum Polymerrückgrat bedeutet.

19. Zusammensetzung, mindestens enthaltend jeweils bezogen auf das Gesamtgewicht der Zusammensetzung
(i) von 0 und 40 Gew.-% einer Gesamtmenge von mindestens einer aromatischen Verbindung mit mindestens zwei Aminogruppen, ,
(ii) zwischen 0 und 35 Gew.-% einer Gesamtmenge von mindestens einer aromatischen Verbindung mit genau einer Aminogruppe, bevorzugt von Anilin,(iii) zwischen 0 und 40 Gew.-% Kohlenwasserstoff-Verbindungen, enthaltend mindestens eine funktionelle Gruppe mit mindestens einem Sauerstoffatomund keine funktionelle Gruppe mit Stickstoffatom,
(iii) von 0 bis 40 Gew.-% einer Gesamtmenge mindestens einer Kohlenwasserstoff-Verbindung, enthaltend mindestens eine funktionelle Gruppe mit mindestens einem Sauerstoffatom und keine funktionelle Gruppe mit Stickstoffatom,
(iv) wobei die Gewichtsanteile innerhalb der Gewichtsanteilbereiche aus (i), (ii) und (iii) derart ausgewählt werden, dass die Summe der gewählten Gewichtanteile aus (i), (ii) und (iii) gemeinsam mit den Gewichtsanteilen weiterer Inhaltsstoffe 100 Gew.-% ergibt.
